# EUROPEAN PATENT SPECIFICATION

(11) **EP 4 329 598 B1**
(45) Date of publication and mention of the grant of the patent: **04.03.2026**
(21) Application number: 22723454.9
(22) Date of filing: 21.04.2022
(51) Int. Cl.: A61B 5/022

(54) **A SHELL CUFF**
SCHALENMANSCHETTE
MANCHON DE COQUE

(30) Priority: 28.04.2021 EP 21171020
(43) Date of publication of application: 06.03.2024
(73) Proprietor: Koninklijke Philips N.V., 5656 AG Eindhoven (NL)
(72) Inventor: AMINE EL SAYED, Achraf, 5656 AG Eindhoven (NL); CNOSSEN, Gerard, 5656 AG Eindhoven (NL)
(74) Representative: Philips Intellectual Property & Standards
(86) International application number: PCT/EP2022/060505
(87) International publication number: WO 2022/228982

(56) References cited:
- EP-A2- 1 566 143
- WO-A1-2014/121805
- WO-A1-2014/125431
- US-A1- 2010 121 205

## Description

### FIELD OF THE INVENTION

The invention relates to a shell cuff for use with an actuator cuff and a sensor pad in hemodynamic monitoring, and a kit for hemodynamic monitoring having such a shell cuff.

### BACKGROUND OF THE INVENTION

US2010121205A1 relates to a blood pressure monitor which is used for an automatic blood pressure monitor, and which includes noise generation inhibiting means for inhibiting the noise caused by the friction of overlapping parts of a cuff to elevate a cuff pressure to a proper pressure value.

Hemodynamic monitoring typically requires invasive techniques or non-invasive monitoring which includes a shell cuff together with an actuator cuff and a sensor pad. Shell cuffs are typically coated with low friction materials to reduce friction between parts in sliding contact. However, coating with low friction materials can be an expensive and time-consuming process.

### SUMMARY OF THE INVENTION

The invention is defined by the independent claims. The dependent claims define advantageous embodiments.

According to a first specific aspect, there is provided a shell cuff for use with an actuator cuff and sensor pad in hemodynamic monitoring, the shell cuff comprising: a flexible sheet comprising a first face and an opposing second face, the sheet being curved such that a first end of the sheet and an opposing second end of the sheet overlap to form an overlapping portion in which a first interface portion of the first face interfaces with a second interface portion of the second face; wherein the second face is defined by a second residual portion and the second interface portion, and wherein the second interface portion is flush with the second residual portion; wherein the sheet is configured to be tightened on application of force so as to increase the size of the overlap between the first face and the second face; wherein the first interface portion is made from a first material and the second interface portion is made from a second material which is different to the first material, and wherein the first material at the first interface portion extends through more than 20% of the thickness of the sheet, and the second material at the second interface portion extends through more than 20% of the thickness of the sheet. The sensor pad is filled with liquid to measure hemodynamic parameter such as blood pressure.

Due to the incompressibility of the fluid in the sensor pad, the measurement of hemodynamic parameter in a shell cuff can be more accurate and more sensitive.

Having different materials in contact which one another lowers the friction in the shell cuff in use, and having layers of material in these proportions enables the layers of the sheet to be easily manufactured by extrusion or injection moulding, such that there is no need to apply a coating of low friction material. This improves the cost efficiency of producing a shell cuff.

The first face may be defined by a first residual portion and the first interface portion.

The respective first interface portion or second interface portion may comprise a different material to the residual portion and the other of the first interface portion and second interface portion.

The sheet may comprise a first layer comprising the first material which forms the first face and a second layer comprising the second material which forms the second face. This enables the sheet to be easily manufactured by extrusion.

One of the first face and second face may comprise a textured surface on at least the respective first interface portion or the second interface portion. This may further reduce the friction between the first interface portion and the second interface portion.

One of the first interface portion and the second interface portion may comprise a compressible material impregnated with silicone oil which is configured to be released on application of pressure to the compressible material. This may further reduce the friction between the first interface portion and the second interface portion.

The first material may be HDPE and the second material may be polyamide which are bonded together with an adhesive layer.

The first material may comprise a first plastic and the second material may comprise the first plastic modified with PTFE particles or silicone oil. These materials may bond together particularly well with an adhesive layer and provide a low friction interface.

The first interface portion may be flush with the first residual portion. The second interface portion is flush with the second residual portion. This reduces the likelihood of snagging of the first face against the second face when they slide relative to one another in contact.

According to a second aspect, there is provided a hemodynamic monitoring kit comprising a shell cuff according to the first aspect, a pressure sensor pad, and an actuator cuff configured to surround the shell cuff and to inflate to apply pressure to the shell cuff.

According to a third aspect, there is provided a method of manufacturing a shell cuff according to the first aspect, the method comprising injection moulding the first material and the second material to form a sheet, and thermoforming the sheet to form the shell cuff.

According to a fourth aspect, there is provided a method of manufacturing a shell cuff according to the first aspect, the method comprising extruding the first material and the second material to form a sheet having at least two extruded layers, and thermoforming the sheet to form the shell cuff.

These and other aspects will be apparent from and elucidated with reference to the embodiments described hereinafter.

### BRIEF DESCRIPTION OF THE DRAWINGS

Exemplary embodiments will now be described, by way of example only, with reference to the following drawings, in which:
Figure 1 schematically shows a hemodynamic monitoring kit;
Figure 2 schematically shows an example shell cuff;
Figures 3A-3B schematically shows portions of further examples of shell cuffs; and
Figure 4 is a flow chart for steps of a method to manufacture a shell cuff.

### DETAILED DESCRIPTION OF EMBODIMENTS

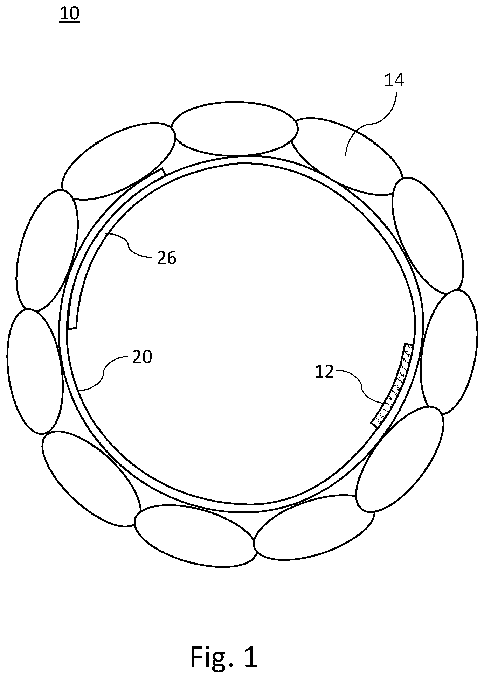

Figure 1 shows a cross-sectional view of an assembled hemodynamic monitoring kit 10. The hemodynamic monitoring kit 10 comprises a sensor pad 12, a shell cuff 20, and an actuator cuff 14.

The sensor pad 12 comprises a sensor configured to sense pressure. The sensor pad 12 can also be filled with liquid to measure hemodynamic parameter such as blood pressure. The shell cuff 20 comprises a flexible sheet which is curved to form a tube such that a first end of the sheet and an opposing second end of the sheet overlap to form an overlapping portion 26. The first end of the sheet and the second end of the sheet are therefore in sliding contact with one another.

The sensor pad 12 is configured to be received on an inside of the tubular shell cuff 20. The actuator cuff 14 is configured to surround the shell cuff 20 and comprises a series of interconnected chambers in a tubular shape which can be inflated to apply pressure to the shell cuff 20.

In use, the assembled hemodynamic monitoring kit 10 is placed around a user's limb with the sensor pad 12 in contact with the limb, and the actuator cuff 14 is inflated to apply pressure to the shell cuff 20 which distributes the pressure evenly around the limb of the user. In some examples, the shell cuff may be rolled into a conical shape to accommodate the shape of a user's limb, such as an arm. Since the shell cuff 20 comprises a flexible sheet, when pressure is applied by the actuator cuff 14, the shell cuff 20 is tightened so that it forms a tube with a smaller diameter, and the size of the overlapping portion 26 increases by relative sliding of the first end of the sheet and the second end of the sheet. Such sliding introduces friction into the system which may disrupt or create noise in the pressure reading of the sensor pad 12. It is therefore desirable to reduce the friction as much as possible.

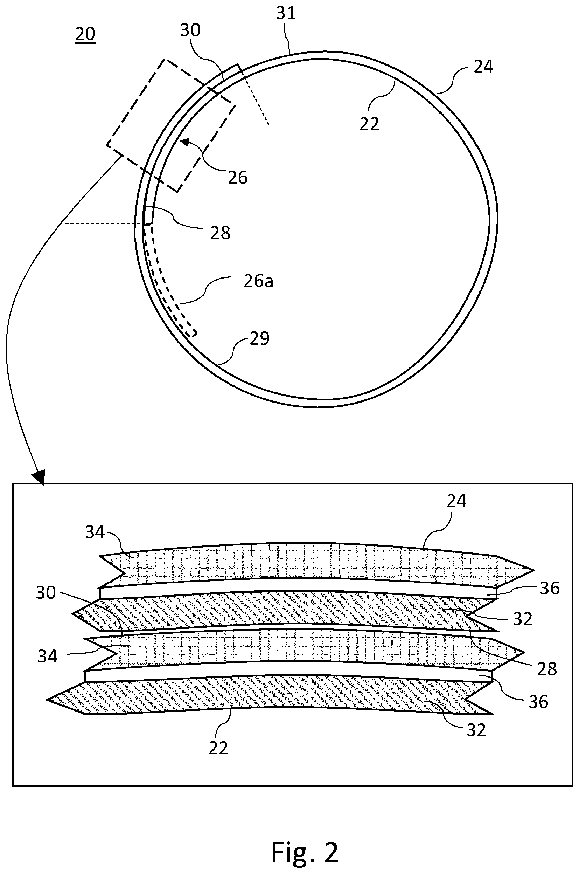

**Figure 2** shows a cross-sectional view of a first example shell cuff 20 together with a close-up view of the overlapping portion 26 of the example shell cuff 20.

The flexible sheet of the shell cuff 20 which is rolled into a tubular shape with the overlapping portion 26 has a memory of the tubular shape, such that it naturally retains this shape. As explained above, the shell cuff 20 can be tightened on application of force, such as from the actuator cuff 14, so as to increase the size of the overlapping portion 26a, shown with dotted lines in Figure 2. The diameter of the tubular shell cuff 20 would also decrease on tightening, but this is not shown in Figure 2 for clarity.

The shell cuff 20 comprises a first face 22 and an opposing second face 24. In this example, the first face 22 is an inner face of the tubular shell cuff 20 and the second face 24 is an outer face of the tubular shell cuff 20. In other words, the first face 22 faces radially inwards from the shell cuff 20 and the second face 24 faces radially outwards from the shell cuff 20. In other examples, the first face may be an outer face and the second face may be an inner face.

As explained above, the shell cuff 20 has a memory of the tubular shape and therefore retains the tubular shape without external force. When there is no external force applied to the shell cuff 20, the first face 22 is defined by a first interface portion 28 and a first residual portion 29 and the second face 24 is defined by a second interface portion 30 and a second residual portion 31, where the first interface portion 28 interfaces with the second interface portion 30 at the overlapping portion 26. The line defining the separation of the respective interface portion and residual portion on the first face 22 and second face 24 are shown with dotted lines cutting the sheet. The first face 22 interfaces with the second face 24 at the overlapping portion 26 due to the curved, tubular shape of the shell cuff 20. When the shell cuff 20 is tightened on application of force, the first interface portion 28 and part of the first residual portion 29 interfaces with the second interface portion 30 and part of the second residual portion 31 due to the overlap increasing.

In this example, the flexible sheet of the shell cuff 20 is formed of a first material 32 in the form of a first layer and a second material 34 in the form of a second layer which are bonded together with a layer of adhesive 36. In this example, the first material 32 forms the first face 22 of the shell cuff 20 and the second material 34 forms the second face 24 of the shell cuff 20. In other examples, the first layer and the second layer may be bonded together without adhesive.

In this example, the first material comprises HDPE and the second material comprises polyamide. In other examples, the first material may comprise polypropylene and the second material may comprise polypropylene modified with PTFE particles or silicone oil. In yet further examples, one of the first or second material may comprise any plastic, and the other of the first or second material may comprise the plastic modified with PTFE particles or silicone oil, or any other low friction material. The first material and the second material may comprise any suitable materials provided at least the material defining the first interface portion is different to the material defining the second interface portion.

Using different materials in the different layers ensures that the first interface portion 28 and the second interface portion 30 comprise different materials. The first interface portion 28 and the second interface portion 30 comprising different materials ensures that, where there is sliding contact between surfaces of the shell cuff 20, van der Waals forces are reduced, thereby reducing friction between the surfaces without the need for an additional coating.

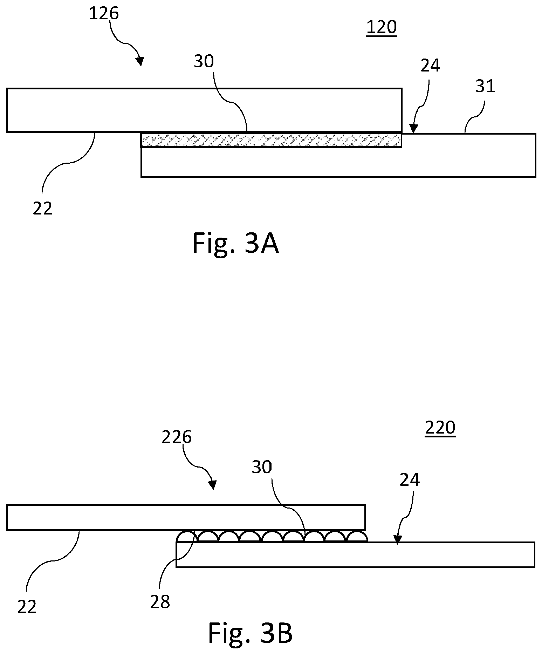

**Figure 3A** shows a cross-sectional view of an overlapping portion 126 of a second example shell cuff 120. **Figure 3B** shows a cross-sectional view of an overlapping portion 226 of a third example shell cuff 220.

The second example shell cuff 120 is similar to the first example shell cuff 20 in comprising a first face 22 and an opposing second face 24 and differs only in the construction of the flexible sheet of the shell cuff 120. In this example, the first face 22 is made from the first material, and the second interface portion 30 is made from the second material. The second residual portion 31 is made from the first material such that only the second interface portion 30 of the second face 24 comprises the second material. In this example, the whole sheet therefore comprises the first material except for a part of the sheet defining the second interface portion 30 of the second face 24.

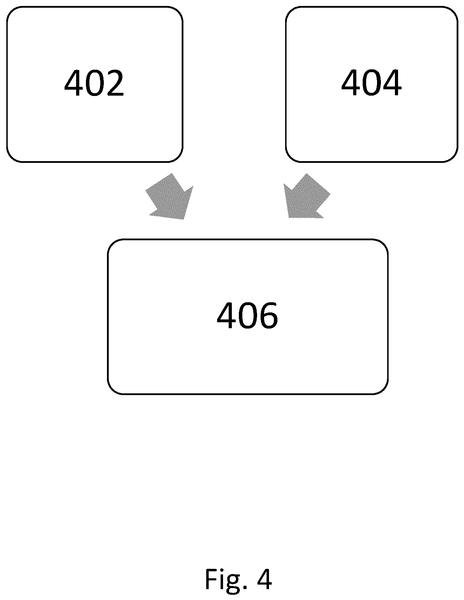

The second interface portion 30 of the second face 24 is flush with the second residual portion 31 of the second face 24. In some examples, the second interface portion may be offset from the second residual portion. In this example, the second material extends through at least one third of the thickness of the sheet. In other examples, the second material may extend through more than 20% of the thickness of the sheet, up to 50% of the thickness or up to the whole thickness of the sheet at the second interface portion. In other words, the sheet at the second interface portion may comprise a thickness of which at least 20% of the thickness comprises the second material (i.e. the ratio of the thickness of the second material to the thickness of the whole sheet at the second interface portion is at least 20%). This can also be applied to the first material at the first interface portion. This may relate to a minimum thickness of the first material and the second material of 1.5mm. This minimum ratio of thicknesses also enables the layers of the sheet to be extruded or injection moulded more easily, as described with reference to Figure 4.

In some examples, the first material and second material may comprise HDPE, LDPE, polyamide or any plastic provided that the first and the second material are different such that at least the first interface portion 28 comprises a different material to the second interface portion 30. In an example in which the second material is a compressible plastic impregnated with silicone oil, on application of pressure, the silicone oil may be released, thereby forming a low friction layer of oil between the first interface portion 28 and the second interface portion 30.

The third example shell cuff 220 is similar to the second example shell cuff 120 and differs in that the second interface portion 30 comprises a textured surface. The textured surface may reduce a contact surface area between the first interface portion 28 and the second interface portion 30 in use, thereby further reducing the friction between the first face 22 and the second face 24.

Although the shell cuffs 120 in Figures 3A-3B have been described as having a second interface portion comprising a second material, with the first face and the second residual portion comprising a first material, in other examples, the second residual portion may comprise a third material which is different from the first material and the second material. For example, the sheet may comprise two layers of the first material and the third material, with only the second interface portion comprising the second material. In further examples, the second material may extend beyond the second interface portion into a part of the second residual portion such that friction is further reduced even when the shell cuff is tightened on application of force. In yet further examples, the first interface portion may comprise the second material with the second face and the first residual portion comprising the first material and/or third material.

**Figure 4** is a flow chart showing steps of a method of forming a shell cuff 20, 120, 220 as described with references to any of the preceding Figures 1-3.

In block 402, the method comprises extruding the first material and the second material to form the sheet of a shell cuff having at least two layers of material, for example the first example shell cuff 20 described with reference to Figure 2.

In block 404, the method comprises injection moulding the first material and the second material to form a sheet as described in any of the examples described herein.

In block 406, the method comprises thermoforming the sheet made in either block 402 or block 404, to form the shell cuff 20, 120, 220 as described in any of the examples described herein.

Injection forming the sheet to form the shell cuff 20, 120, 220 enables the textures to be added to the first or second faces of the sheet, and enables a portion of a face to comprise a different material to the rest of the face, such as described in the second and third example shell cuffs. Both extruding and injection moulding the materials to form a shell cuff having two different materials at an overlap of the shell cuff means that the effect of reduced friction is achieved without requiring a coating for the sheet, thereby providing a cost-effective method of manufacturing a reduced friction shell cuff.

## Claims

1. A shell cuff (20, 120, 220) for use with an actuator cuff (14) and sensor pad (12) in hemodynamic monitoring, the shell cuff (20, 120, 220) comprising:
a flexible sheet comprising a first face (22) and an opposing second face (24), the sheet being curved such that a first end of the sheet and an opposing second end of the sheet overlap to form an overlapping portion (26, 26a, 126, 226) in which a first interface portion (28) of the first face (22) interfaces with a second interface portion (30) of the second face (24);
wherein the second face is defined by a second residual portion (31) and the second interface portion, and wherein the second interface portion is flush with the second residual portion;
wherein the sheet is configured to be tightened on application of force so as to increase the size of the overlap between the first face (22) and the second face (24);
wherein the first interface portion (28) is made from a first material (32) and the second interface portion (30) is made from a second material (34) which is different to the first material (32), and wherein the first material at the first interface portion (28) extends through more than 20% of the thickness of the sheet, and the second material at the second interface portion (30) extends through more than 20% of the thickness of the sheet, and
wherein the sensor pad is filled with liquid to measure a hemodynamic parameter.

2. A shell cuff (20, 120, 220) according to claim 1, wherein the first face (22) is defined by a first residual portion (29) and the first interface portion (28), and wherein the respective first interface portion (28) or second interface portion (30) comprises a different material to the residual portion (29, 31) and the other of the first interface portion (28) and second interface portion (30).

3. A shell cuff (20, 120, 220) according to claim 1, wherein the sheet comprises:
a first layer comprising the first material (32) which forms the first face (22); and
a second layer comprising the second material (34) which forms the second face (24).

4. A shell cuff (20, 120, 220) according to any preceding claim, wherein one of the first face (22) and second face (24) comprises a textured surface on at least the respective first interface portion (28) or the second interface portion (30).

5. A shell cuff (20, 120, 220) according to any preceding claim, wherein one of the first interface portion (28) and the second interface portion (30) comprises a compressible material impregnated with silicone oil which is configured to be released on application of pressure to the compressible material.

6. A shell cuff (20, 120, 220) according to any preceding claim, wherein the first material (32) is HDPE, and the second material (34) is polyamide which are bonded together with an adhesive (36) layer.

7. A shell cuff (20, 120, 220) according to any of claims 1-5, wherein the first material (32) comprises a first plastic and the second material (34) comprises the first plastic modified with PTFE particles or silicone oil.

8. A shell cuff (20, 120, 220) according to any preceding claim, wherein the first face (22) is defined by a first residual portion (29) and the first interface portion (28), and wherein the first interface portion (29) is flush with the first residual portion (29).

9. A hemodynamic monitoring kit comprising:
a shell cuff (20, 120, 200, 220) according to any preceding claim;
a pressure sensor pad (12); and
an actuator cuff (14) configured to surround the shell cuff (20, 120, 200, 220) and to inflate to apply pressure to the shell cuff (20, 120, 200, 220).

10. A method of manufacturing a shell cuff (20, 120, 200, 220) according to any of claims 1-9, the method comprising injection moulding the first material (32) and the second material (34) to form a sheet, and thermoforming the sheet to form the shell cuff (20, 120, 200, 220).

11. A method of manufacturing a shell cuff (20, 120, 200, 220) according to claim 3 or any of claims 4-7 when appendant to claim 3, the method comprising extruding the first material (32) and the second material (34) to form a sheet having at least two extruded layers, and thermoforming the sheet to form the shell cuff (20, 120, 200, 220).

## Patentansprüche

1. Schalenmanschette (20, 120, 220) zur Verwendung mit einer Betätigungsmanschette (14) und einem Sensorkissen (12) bei der hämodynamischen Überwachung, wobei die Schalenmanschette (20, 120, 220) Folgendes umfasst:
eine flexible Folie, die eine erste Fläche (22) und eine gegenüberliegende zweite Fläche (24) umfasst, wobei die Folie so gekrümmt ist, dass sich ein erstes Ende der Folie und ein gegenüberliegendes zweites Ende der Folie überlappen und einen Überlappungsbereich (26, 26a, 126, 226) bilden, in dem ein erster Grenzflächenbereich (28) der ersten Fläche (22) mit einem zweiten Grenzflächenbereich (30) der zweiten Fläche (24) in Kontakt steht;
wobei die zweite Fläche durch einen zweiten Restabschnitt (31) und den zweiten Grenzflächenabschnitt definiert ist und wobei der zweite Grenzflächenabschnitt bündig mit dem zweiten Restabschnitt abschließt;
wobei die Folie so konfiguriert ist, dass sie sich bei Krafteinwirkung spannt, um die Größe der Überlappung zwischen der ersten Fläche (22) und der zweiten Fläche (24) zu vergrößern;
wobei der erste Grenzflächenabschnitt (28) aus einem ersten Material (32) besteht und der zweite Grenzflächenabschnitt (30) aus einem zweiten Material (34) besteht, das sich von dem ersten Material (32) unterscheidet, und wobei sich das erste Material im ersten Grenzflächenabschnitt (28) über mehr als 20 % der Foliendicke erstreckt und das zweite Material im zweiten Grenzflächenabschnitt (30) sich über mehr als 20 % der Foliendicke erstreckt, und
wobei das Sensorkissen mit Flüssigkeit gefüllt ist, um einen hämodynamischen Parameter zu messen.

2. Schalenmanschette (20, 120, 220) nach Anspruch 1, wobei die erste Fläche (22) durch einen ersten Restabschnitt (29) und den ersten Grenzflächenabschnitt (28) definiert ist und wobei der jeweilige erste Grenzflächenabschnitt (28) bzw. zweite Grenzflächenabschnitt (30) ein anderes Material als der Restabschnitt (29, 31) und der andere von dem ersten Grenzflächenabschnitt (28) und dem zweiten Grenzflächenabschnitt (30) umfasst.

3. Schalenmanschette (20, 120, 220) nach Anspruch 1, wobei die Folie Folgendes umfasst:
eine erste Schicht, die ein erstes Material (32) umfasst, das die erste Fläche (22) bildet; und
eine zweite Schicht, die ein zweites Material (34) umfasst, das die zweite Fläche (24) bildet.

4. Schalenmanschette (20, 120, 220) nach einem vorstehenden Anspruch, wobei eine von der ersten Fläche (22) und der zweiten Fläche (24) mindestens auf dem jeweiligen ersten Grenzflächenabschnitt (28) bzw. dem zweiten Grenzflächenabschnitt (30) eine strukturierte Oberfläche umfasst.

5. Schalenmanschette (20, 120, 220) nach einem vorstehenden Anspruch, wobei einer von dem ersten Grenzflächenabschnitt (28) und dem zweiten Grenzflächenabschnitt (30) ein kompressibles Material umfasst, das mit Silikonöl imprägniert ist und so konfiguriert ist, dass es bei Druckeinwirkung auf das kompressible Material freigesetzt wird.

6. Schalenmanschette (20, 120, 220) nach einem vorstehenden Anspruch, wobei das erste Material (32) HDPE ist und das zweite Material (34) Polyamid ist, die mit einer Klebeschicht (36) miteinander verbunden sind.

7. Schalenmanschette (20, 120, 220) nach einem der Ansprüche 1-5, wobei das erste Material (32) einen ersten Kunststoff umfasst und das zweite Material (34) den ersten Kunststoff, modifiziert mit PTFE-Partikeln oder Silikonöl, umfasst.

8. Schalenmanschette (20, 120, 220) nach einem vorstehenden Anspruch wobei die erste Fläche (22) durch einen ersten Restabschnitt (29) und den ersten Grenzflächenabschnitt (28) definiert ist und wobei der erste Grenzflächenabschnitt (29) bündig mit dem ersten Restabschnitt (29) abschließt.

9. Kit zur hämodynamischen Überwachung, umfassend:
eine Schalenmanschette (20, 120, 200, 220) nach einem vorstehenden Anspruch;
ein Drucksensorkissen (12); und
eine Betätigungsmanschette (14), die so konfiguriert ist, dass sie die Schalenmanschette (20, 120, 200, 220) umschließt und sich aufbläht, um Druck auf die Schalenmanschette (20, 120, 200, 220) auszuüben.

10. Verfahren zur Herstellung einer Schalenmanschette (20, 120, 200, 220) nach einem der Ansprüche 1-9, wobei das Verfahren das Spritzgießen des ersten Materials (32) und des zweiten Materials (34) zum Bilden einer Folie und das Thermoformen der Folie zum Bilden der Schalenmanschette (20, 120, 200, 220) umfasst.

11. Verfahren zur Herstellung einer Schalenmanschette (20, 120, 200, 220) nach Anspruch 3 oder einem der Ansprüche 4-7, wenn diese dem Anspruch 3 beigefügt sind, wobei das Verfahren das Extrudieren des ersten Materials (32) und des zweiten Materials (34) zum Bilden einer Folie mit mindestens zwei extrudierten Schichten und das Thermoformen der Folie zum Bilden der Schalenmanschette (20, 120, 200, 220) umfasst.

## Revendications

1. Manchon de coque (20, 120, 220) destiné à être utilisé avec un manchon d'actionneur (14) et un coussinet de capteur (12) pour la surveillance hémodynamique, le manchon de coque (20, 120, 220) comprenant :
une feuille flexible comprenant une première face (22) et une seconde face opposée (24), la feuille étant courbée de telle sorte qu'une première extrémité de la feuille et une seconde extrémité opposée de la feuille se chevauchent pour former une portion de chevauchement (26, 26a, 126, 226) dans laquelle une première portion d'interface (28) de la première face (22) s'interface avec une seconde portion d'interface (30) de la seconde face (24) ;
dans lequel la seconde face est définie par une seconde portion résiduelle (31) et la seconde portion d'interface, et dans lequel la seconde portion d'interface est alignée avec la seconde portion résiduelle ;
dans lequel la feuille est configurée pour être serrée lors de l'application d'une force afin d'augmenter la taille du chevauchement entre la première face (22) et la seconde face (24) ;
dans lequel la première portion d'interface (28) est constituée d'un premier matériau (32) et la seconde portion d'interface (30) est constituée d'un second matériau (34) qui est différent du premier matériau (32), et dans lequel le premier matériau au niveau de la première portion d'interface (28) s'étend sur plus de 20 % de l'épaisseur de la feuille, et le second matériau au niveau de la seconde portion d'interface (30) s'étend sur plus de 20 % de l'épaisseur de la feuille, et
dans lequel le coussinet de capteur est rempli de liquide pour mesurer un paramètre hémodynamique.

2. Manchon de coque (20, 120, 220) selon la revendication 1, dans lequel la première face (22) est définie par une première portion résiduelle (29) et la première portion d'interface (28), et dans lequel la première portion d'interface respective (28) ou la seconde portion d'interface (30) comprend un matériau différent de la portion résiduelle (29, 31) et de l'autre de la première portion d'interface (28) et de la seconde portion d'interface (30).

3. Manchon de coque (20, 120, 220) selon la revendication 1, dans laquelle la feuille comprend :
une première couche comprenant le premier matériau (32) qui forme la première face (22) ; et
une seconde couche comprenant le second matériau (34) qui forme la seconde face (24).

4. Manchon de coque (20, 120, 220) selon une quelconque revendication précédente, dans lequel l'une de la première face (22) et de la seconde face (24) comprend une surface texturée sur au moins la première portion d'interface respective (28) ou la seconde portion d'interface (30).

5. Manchon de coque (20, 120, 220) selon une quelconque revendication précédente, dans lequel l'une de la première portion d'interface (28) et de la seconde portion d'interface (30) comprend un matériau compressible imprégné d'huile de silicone qui est configuré pour être libéré lors de l'application d'une pression sur le matériau compressible.

6. Manchon de coque (20, 120, 220) selon une quelconque revendication précédente, dans lequel le premier matériau (32) est du PEHD et le second matériau (34) est du polyamide qui sont liés ensemble par une couche adhésive (36).

7. Manchon de coque (20, 120, 220) selon l'une quelconque des revendications 1-5, dans lequel le premier matériau (32) comprend un premier plastique et le second matériau (34) comprend le premier plastique modifié avec des particules de PTFE ou de l'huile de silicone.

8. Manchon de coque (20, 120, 220) selon une quelconque revendication précédente, dans lequel la première face (22) est définie par une première portion résiduelle (29) et la première portion d'interface (28), et dans lequel la première portion d'interface (29) est alignée avec la première portion résiduelle (29).

9. Kit de surveillance hémodynamique comprenant :
un manchon de coque (20, 120, 200, 220) selon une quelconque revendication précédente ;
un coussinet de capteur de pression (12) ; et
un manchon d'actionneur (14) configuré pour entourer le manchon de coque (20, 120, 200, 220) et pour se gonfler afin d'appliquer une pression sur le manchon de coque (20, 120, 200, 220).

10. Procédé de fabrication d'un manchon de coque (20, 120, 200, 220) selon l'une quelconque des revendications 1-9, le procédé comprenant le moulage par injection du premier matériau (32) et du second matériau (34) pour former une feuille, et le thermoformage de la feuille pour former le manchon de coque (20, 120, 200, 220).

11. Procédé de fabrication d'un manchon de coque (20, 120, 200, 220) selon la revendication 3 ou l'une quelconque des revendications 4-7 lorsqu'elle est annexée à la revendication 3, le procédé comprenant l'extrusion du premier matériau (32) et du second matériau (34) pour former une feuille présentant au moins deux couches extrudées, et le thermoformage de la feuille pour former le manchon de coque (20, 120, 200, 220).
